# EUROPEAN PATENT SPECIFICATION

(11) **EP 2 114 317 B1**
(45) Date of publication and mention of the grant of the patent: **10.08.2016**
(21) Application number: 08701829.7
(22) Date of filing: 18.01.2008
(51) Int. Cl.: A61F 2/68, A61F 2/78, A61F 2/28

(54) **TENDON-INTEGRATED PROSTHESIS**
PROTHESE MIT SEHNENANBINDUNG
PROTHÈSE INTÉGRÉE À UN TENDON

(30) Priority: 18.01.2007 GB 0700975
(43) Date of publication of application: 11.11.2009
(73) Proprietor: UCL Business PLC, London W1T 4TP (GB)
(72) Inventor: BLUNN, Gordon, Stanmore Middlesex HA7 4LP (GB); UNWIN, Paul, Stanmore Middlesex HA7 4LP (GB); KANG, Norbert, Middlesex HA6 2YR (GB); LOW, Nigel, Middlesex HA7 4LP (GB); PENDEGRASS, Catherine, Middlesex HA7 4LP (GB)
(74) Representative: Maughan, Sophie Louise
(86) International application number: PCT/GB2008/000151
(87) International publication number: WO 2008/087412

(56) References cited:
- EP-A- 1 438 937
- WO-A-02/38083
- DE-A1- 1 800 112
- DE-C1- 19 845 191
- US-A- 3 971 670
- US-A- 4 143 426

## Description

### Field of the Invention

The present invention relates to a tendon-integrated prosthetic device for functional reconstruction of amputated limb extremities (i.e., digits, hands, feet, arms and legs).

### Background to the Invention

The majority of existing limb extremity prostheses are not directly linked to internal musculature in the residual limb and often are essentially cosmetic, being static and non-functional or may in some cases be functional but limited in capability.

The standard external prosthesis in the upper limb (especially the hand) is usually rigid and non-functional. It is mainly used to improve the cosmetic appearance of the amputated part. When used for reconstruction of an amputated part (e.g. the hand) it is often difficult to secure and may slip off at awkward moments. In the lower limb, a standard prosthesis is more functional since it is weight-bearing and articulated. However, the function may still be impaired by the lack of any secure connection to the residual tissues.

Body-powered prostheses linked by external cables are available and are more useful, being usually of moderate cost and weight, but are only suitable for the upper limb. They are the most durable prostheses and have higher sensory feedback. However, such body-powered prostheses are less cosmetically pleasing than a myoelectric unit and they require gross limb movements to activate the prosthesis.

The myoelectric prosthesis is an external prosthesis that is linked to the residual musculature. The prosthesis is linked to the residual muscles through an external electrode placed on the skin. These electrodes pick up the faint electrical signal produced by voluntary contractions of the residual muscles. This activates an electrically powered external prosthesis. However, the link is crude and the movements which can be produced are therefore relatively crude. The link is also unreliable because the cutaneous electrodes can be dislodged. This makes the prosthesis heavy and difficult to use. Prostheses operated by myoelectricity may give more proximal function and increased cosmesis, but they also have less sensory feedback and require more maintenance. There are generally two types of myoelectric prosthesis The 2-site/2-function device has separate electrodes for flexion and extension and the 1-site/2-function device has one electrode for both flexion and extension. The patient uses muscle contractions of different strengths to differentiate between flexion and extension. For example, a strong contraction opens the device, and a weak contraction closes it.

For an upper limb prosthesis, the principle aim is to replicate the function of the hand and to this end terminal devices have been developed. The terminal device prosthesis tries to replicate the 5 different types of grip including: precision grip (i.e. pinch grip); tripod grip (i.e. chuck pinch); lateral grip (e.g. turning a key in a lock); hook power grip (e.g. carrying a briefcase by the handle); and spherical grip (e.g. screwing in a light bulb). This is achieved using active or passive devices.

Passive terminal devices have superior cosmetic appearance but are less functional and more expensive than active terminal devices. Active terminal devices include hooks and prosthetic hands powered by cables or myoelectric devices and generally have either a voluntary opening mechanism or a voluntary closing mechanism. With a voluntary opening mechanism, the terminal device is closed at rest. The patient uses the proximal muscles to open the device against the resistive force of rubber bands or cables. Relaxation of the proximal muscles allows the terminal device to close around the desired object. If the terminal device is myoelectric, contraction of the proximal muscles activates the electric motor. With a voluntary closing mechanism, the terminal device is open at rest and the patient uses the residual forearm flexors to grasp the desired object. This type of mechanism is normally heavier and less durable than a voluntary opening mechanism and hence is less common.

All the terminal devices currently available represent significant compromises in terms of appearance and function. Existing external prostheses are generally not directly linked to internal musculature in the residual limb. Indirect linkage to internal musculature is inefficient and does not allow for sophisticated movements to be performed by the external prosthesis. Furthermore, the indirect provision of motive force for an external prosthesis uses unsightly cables or cumbersome electronics. Leading on from these problems in the art there is no, functional, upper limb prosthesis for reconstruction of very distal amputations (e.g. amputation of the fingers) and there is no lower limb prosthesis which provides the same level of function as a cable or myoelectric driven upper limb prosthesis.

One known lower limb prosthesis improves over this art in a limited way by having a tendon-integrated arrangement. This is disclosed in US patent US 4,143,426 (Hall), the contents of which are incorporated herein in their entirety by reference. In this prosthesis an artificial tendon comprising a seine cord of nylon is ensheathed in a velour sleeve and sutured to the native tendon or muscle. A velour-covered Silastic ball is integral with or attached to the artificial tendon at the location where the artificial tendon passes through the skin to provide a strong skin interface even under the stresses of external loading. The external part or external extension of the artificial tendon externally attaches to a lever part of a bone-implanted external articulating mechanical joint. However, the prosthesis relies on a considerable extent of skin-enveloped free muscle/tendon in a stump that extends alongside the bone stump of the amputated upper limb in order that flexure of the muscle/tendon may move the articulating mechanical joint. This system has much of the disadvantage of the afore-described external-cable linked body-powered prostheses, cannot be used for many patients and is unsuitable for upper limb prostheses and terminal devices.

DE 198 45 191 C1 discloses a leg prosthesis comprising an adaptor 2 for a knee joint 3. The adaptor has a proximal rod section 7 which is implantable in an upper leg stump 1. In order to transmit forces generated by muscles in said stump to the knee joint, a bridle 5 and a stirrup 6 are provided external on the lower part 13 of the knee joint. A skin-lined tunnel is formed in the muscle of the stump, through which a pin forming a cross-bar for the stirrup is installed linking the stirrup via the bridle to the prosthesis.

Amongst other objectives the present invention seeks to provide a prosthetic device that addresses one or more of the foregoing drawbacks of the art, suitably enabling reconstruction of very distal amputations (eg amputation of fingers); enabling closer reproduction of a normal limb's complex movement capability in an external prosthesis used for limb reconstruction after amputation; enabling use of the residual limb muscles to provide the motive force for the external prosthesis; and providing the potential for neural feedback from the tendons and muscles in the residual limb.

### Summary of the Invention

According to a first aspect of the present invention there is provided a transcutaneous prosthetic device which comprises:
(i) a or at least one soft tissue fixture adapted to be fixed to the musculo-tendinous soft tissues of a residual limb;
(ii) a transcutaneous anchor for an external prosthesis that is fixedly coupled, directly or indirectly, to the bone of the residual limb in use, the transcutaneous anchor having or being rigidly coupled to a transcutaneous component having a body which passes through the skin in use;
(iii) at least one transmission means, at least part of the transmission means being housed, in use, within said body of the transcutaneous component, the transmission means allowing transmission of one or more signals between the soft tissue fixture and an external prosthesis, the signal or signals relating to contraction and/or relaxation of muscle in the residual limb, whereby the external prosthesis can be controlled by contraction and/or relaxation of muscle in the residual limb.

Preferably the transmission means comprises a connecting means connecting the soft tissue fixture and an external prosthesis in use, at least part of the connecting means being housed, in use, within a body of the transcutaneous component, whereby the external prosthesis can be controlled via the connecting means, by contraction and/or relaxation of muscle in the residual limb.

The connecting means may comprises a mechanical or an electrical connector. Thereby, the transmission of signals (containing information as to contraction and/or relaxation of muscle in the residual limb) to the external prosthesis may be mechanical or electrical. The device may include two or more soft tissue fixtures connected to the external prosthetic device via corresponding connecting means.

Preferably the connecting means comprises a sealed connection within the transcutaneous component, to provide an effective barrier between the internal and external environments.

Preferably the connecting means comprises a connector attached, in use, by its proximal end to the fixture and which allows the mechanical force of muscle contraction to be transmitted to an external prosthesis, the transcutaneous anchor having or being rigidly/fixedly coupled to a transcutaneous sleeve component into which the connector extends subcutaneously and from which the connector or an extension thereof extends externally from the skin, the sleeve component forming a fluid tight barrier, the subcutaneous part of the connector being slidable back and forth into the transcutaneous sleeve component whereby the external part or extension of the connector correspondingly moves back and forth.

In a preferred embodiment the sleeve component further comprises a rotary member and the connector is attached at its distal end to the rotary member to rotate the rotary member when the connector moves back and forth, a or the fluid tight barrier being formed around the rotary member, the movement of the connector being transmitted beyond the sleeve by an extension connector attached to the rotary member, or an extension thereof, on the external side of the barrier.

In an alternative embodiment the sleeve component further comprises an extendible and collapsible envelope/ sheath that envelops the external part or extension of the connector.

Preferably the connector comprises a cable or cord the surface of which has a non-stick coating or is otherwise adapted to deter tissue adhesion and minimise friction with the surrounding tissues. The connector may be housed in a sheath for a substantial part of its length.

In an alternative embodiment the transmission means or connecting means comprises an electrical connector, the connecting means or the fixture including a sensor which generates an electrical signal in response to contraction and/or relaxation of muscle in the residual limb. The sensor may comprise a potentiometer or a strain gauge.

Preferably the transcutaneous component has at least one feed-through connection into which the electrical connector or an extension thereof extends subcutaneously and from which the electrical connector or an extension thereof extends externally from the skin in use, the feed-through connection being sealed to provide an effective barrier between the internal and external environments.

Preferably the sensor is connectable, via a sealed connection within a body of the transcutaneous component, to a battery in an external prosthesis. Preferably the sensor is also connectable, via a sealed connection within a body of the transcutaneous component, to at least one electric motor adapted to drive movement of the external prosthesis. Suitably the sealed connection comprises a sealed feed-through connection.

The transcutaneous component may comprise at least one electrical contact on a subcutaneous surface of the transcutaneous component, to which said at least one electrical connector and said at least one sensor can be electrically coupled. The transcutaneous component may comprise at least one electrical contact on an extracutaneous surface of the transcutaneous component, to which said at least one electrical connector and an electrical connector of an external device can be electrically coupled. Subcutaneous and/or extracutaneous contacts may also be provided for connection of the sensor to a battery in the external prosthesis.

According to a further aspect of the invention there is provided a transcutaneous prosthetic device which comprises:
(i) a or at least one soft tissue fixture adapted to be fixed to the musculo-tendinous soft tissues of a residual limb;
(ii) a connector attached by its proximal end to the fixture and which allows the mechanical force of muscle contraction to be transmitted to an external prosthesis; and
(iii) a transcutaneous anchor for an external prosthesis that is fixedly coupled, directly or indirectly, to the bone of the residual limb in use, the anchor having or being rigidly/fixedly coupled to a transcutaneous sleeve component into which the connector extends subcutaneously and from which the connector or an extension thereof extends externally from the skin, the sleeve component forming a fluid tight barrier, the subcutaneous part of the connector being slidable back and forth into the transcutaneous sleeve component whereby the external part or extension of the connector correspondingly moves back and forth.

According to a yet further aspect of the invention there is provided a transcutaneous prosthetic device which comprises:
(i) at least one soft tissue fixture adapted to be fixed to the musculo-tendinous soft tissues of a residual limb;
(ii) a transcutaneous anchor for an external prosthesis that is fixedly coupled, directly or indirectly, to the bone of the residual limb in use, the transcutaneous anchor having a transcutaneous component;
(iii) at least one electrical connector connecting the fixture and an external prosthesis in use, at least part of the electrical connector being housed, in use, within a body of the transcutaneous component, the fixture including a sensor which generates an electrical signal in response to contraction and/or relaxation of muscle in the residual limb.

Preferably the soft tissue fixture is adapted to be integrated into the soft tissue, being of a material or having a surface treatment to encourage in-growth of collagen fibres into it.

Preferably the transcutaneous anchor comprises a fixture that is shaped for implantation into bone and treated to stimulate bone growth and osseous integration.

Preferably the transcutaneous anchor has a stem that projects through the skin in use. Suitably the stem is surface treated and/or coated to allow cutaneous integration of the anchor's surface with the skin and preferably the surface of the stem that is external of the skin in use is of very low surface energy to deter bacterial adhesion.

Preferably an external part of the transcutaneous component has a coupling feature for attachment of an external prosthesis.

### Brief Description of the Drawings

Preferred embodiments of the present invention will now be more particularly described, by way of example, with reference to the accompanying drawings in which:
Figure 1 is a perspective view of an index finger amputee's hand with a superimposed schematic diagram illustrating a first preferred embodiment of index finger prosthesis minus the external terminal device of the prosthesis but from which the internal and transcutaneous parts and their operative inter-relationship are apparent;
Figure 2 is a detail view, side elevation, of the tendon-integrated fixture of the internal part of the prosthesis;
Figure 3 is a detail view, side elevation, of the transcutaneous part of the prosthesis;
Figure 4 is a front elevation external view of the transcutaneous part of the prosthesis;
Figure 5 is a detail view, side elevation, of the transcutaneous part of a second embodiment of the prosthesis;
Figure 6 is a front elevation external view of the transcutaneous part of the second embodiment of prosthesis;
Figure 7 is a perspective view of an index finger amputee's hand with a superimposed schematic diagram illustrating a third preferred embodiment of index finger prosthesis and with the external terminal device of the prosthesis shown.
Figure 8 is a side elevation schematic diagram illustrating a fourth preferred embodiment of a finger prosthesis attached to the stump of an amputated finger, showing flexion of the finger prosthesis having two artificial tendons;
Figure 9 is a side elevation schematic diagram of the finger prosthesis of Figure 8, showing extension of the finger prosthesis;
Figure 10 is a side elevation schematic diagram illustrating a fifth embodiment of a finger prosthesis attached to the stump of an amputated finger, showing attachment of artificial tendons to a potentiometer or load cell for flexion/extension of the finger prosthesis using electrical signals.

### Detailed Description of Preferred Embodiments

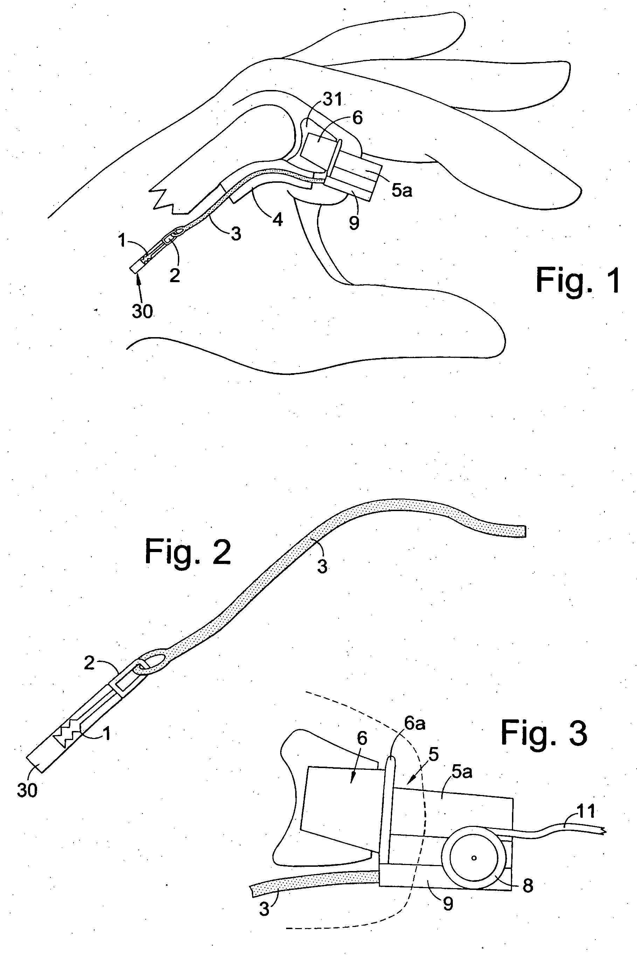

Referring firstly to Figures 1 and 2, these show the first preferred embodiment of the device, which comprises three main components. The first of these components is a tendon-integrated fixture 1. This is inserted or sutured into the native tendon 30. This fixture 1 is adapted to integrate with the collagen substance of the tendon in such a way as to become firmly fixed or embedded within it because the material of the fixture or a surface treatment of the fixture encourages in-growth of the collagen fibres into it and suitably the material is also selected or treated to not be rejected by the tendon substance.

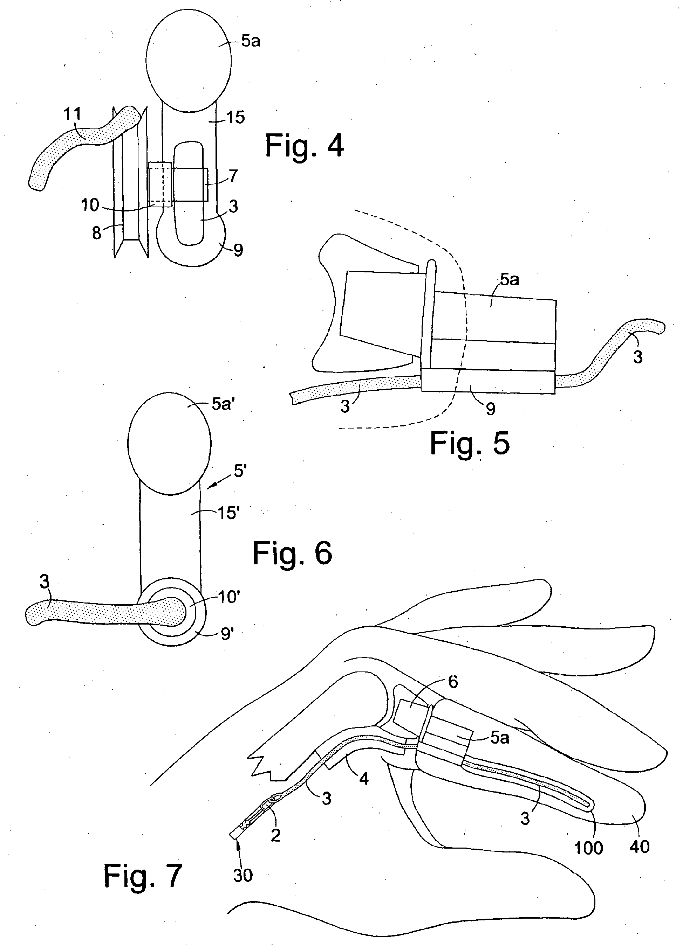

The tendon-fixture 1 has an eyelet 2 or other attachment feature which projects from the end of the tendon. This allows the attachment of a connector 3, that functions as an artificial tendon and which is hereshown as a cable or cord (such as a nylon cord), linking with the external prosthesis 40. The external prosthesis 40, shown in Figure 7, comprises a terminal device corresponding to the missing end of the patient's index finger. Unlike the main body of the tendon fixture 1, the surface of the internal connector 3 cable, and suitably also eyelet 2, is configured to deter soft tissue incorporation, preventing adhesion formation since it needs to remain free for reciprocating movement with the muscle/ native tendon 30. A non-stick coating may be applied to the surface of the internal connector 3 cable to assist this. Example coatings include fluoro- or silicone polymers or diamond-like carbon coatings. Protection of the tissues from abrasion by the connector 3 cable is further provided by a flexible sheath 4 through which the cable 3 extends and which may be adapted to bond with the surrounding tissues.

A further major component of the device is a transcutaneous anchor 5 for anchoring the external prosthesis 40 to the patient's bone. The transcutaneous anchor 5 may be suitably formed of titanium or other metal or metal alloy suitably case to the required shape. The transcutaneous anchor 5 comprises a bone-integrated fixture 6, or is fixedly attached to/ integrally assembled with a bone-integrated fixture 6, which provides a solid anchorage for the external prosthesis 40. The bone-integrated fixture 6 is suitably shaped for implantation into the bone 31, eg tapered and suitably adapted to resist rotation in the bone by, for example, having longitudinal ridges, flutes or other surface features that mesh with a socket in the bone that the fixture 6 is inserted into. Surface treatment of the surface of the bone-integrated fixture with hydroxyapatite (hydrated calcium phosphate) or alumina oxide ceramics will help to stimulate bone growth and osseous integration. The surface may also be formed with small apertures or pits to encourage integration between the bone and the bone-integrated fixture 6. Where micro pits are formed in the surface, these may be of the order of 20 to 500 microns in size, preferably 20 to 100 microns.

A stem 5a of the transcutaneous anchor 5 that projects through the skin is surface-treated/ coated to allow cutaneous-integration of the anchor's surface with the skin. Suitably the surface treatment serves to stimulate fibroblastic cell proliferation and attachment of epithelial cells and may, for example, comprise provision of a porous surface or a surface that is micro-pitted with micro-pits that are of the order of 20 to 500 microns in diameter. The surface treatment may comprise a coating of a material such as hydrated calcium phosphate, alumina oxide ceramics or adhesion promoting proteins such as, for example, fibronectin or laminin. As illustrated, the transition from the bone-integrated conically tapered part 6 to the stem 5a of the anchor 5 is in the form of an annular flange 6a that may serve as an abutment to the end of the bone for additional strength/stability but also may further assist in cutaneous integration if perforated or coated with the treatments for cutaneous integration referred to above. Immediately beyond the skin surface the external surface of the anchor 5 suitably is of a very low surface energy to deter cell adhesion and thereby deter microbes, preferably having a non-stick surface coating such as of fluoro- or silicon polymer or diamond-like carbon. Any such coating may be applied by known techniques such as by chemical vapour deposition (CVD). The external part of the transcutaneous prosthesis suitably terminates in a coupling formation that is shaped to couple to an external prosthesis.

From Figures 3 and 4 it will further be appreciated that the stem 5a of the anchor 5 has a fin 15 projecting from its side running alongside its length and which rigidly carries a rigid tubular sleeve 9 into which the cable 3 extends subcutaneously and in which the cable 3 is able to slide back and forth. The outermost/ distal end of the sleeve 9 is closed / blind and thus the connector cable 3 does not extend beyond it. The connector cable 3 is instead arranged to transmit the force of contraction of the residual muscles from the tendon-fixture 1 to the external prosthesis 40 indirectly.

The cable 3 is, within the sleeve 9, connected to an axle 7 of a small wheel 8 that is positioned on the outer wall of the sleeve 9. The wheel 8 has attached to it one end of an extension cable 11 that may be wound onto or unwound from the wheel 8 as the wheel 8 rotates and the extension cable 11 is attached to the external prosthesis 40. Thus the linear movement of the tendon 30 is translated to rotary movement of the axle 7 and thence the wheel 8 and the rotary movement of the wheel 8 is then converted back to linear movement of the extension cable 11 which leads to the desired movement of the external prosthesis 40.

The internal and external environments of the residual limb are sealed from each other by a seal 10 around the axle 7. This seal 10 is adapted to serve as a barrier to . prevent solid contaminants and air or other fluids from being able to pass from the external to the internal environment by travelling along the axle 7 while still allowing free rotary movement of the axle. Though the axle 7 rotates it does not slide in and out of the barrier. This prevents bacteria and other microbes from entering the internal environment to cause infection of the patient or the prosthesis. As will be appreciated, the present invention thus provides means by which a physical connection to the external prosthesis is maintained while ensuring that the internal environment of the residual limb remains effectively sealed from the external environment.

Sealing off the internal and external environments is critical to prevent ascending infection from entering the residual limb. Failure of the seal could result in ascending infection entering the residual limb, which might lead to serious harm for the amputee and loss of the prosthesis. Creating a permanent and safe physical link between the tendons/muscles in the residual limb and the external environment allows engineers to take a radically new approach to the design of the external prosthesis. Not only will the external prosthesis be able to mimic more of the normal range of movements of the lost part but it makes possible harnessing of the natural neural feedback which is available in the residual limb tendons/muscles.

In a second embodiment of the invention illustrated in Figures 5 and 6, the connector cable 3 transmitting the force from the tendon fixture 1 passes directly through the sleeve 9' of the transcutaneous anchor 5' (Figures 5 and 6). The barrier between the internal and external environments is maintained by a special seal 10' that surrounds the cable as it passes through the transcutaneous anchor. This seal 10' is adapted to prevent air or fluids from being able to pass from the external to the internal environment along the cable 3 as it moves in and out. This seal prevents bacteria and other organisms from entering the internal environment to cause infection of the patient or the prosthesis. The cable is linked directly to the external prosthesis.

A third embodiment, shown in Figure 7, has the form of a direct linkage that extends the seal around the cable into the extended prosthetic digit (Figure 7). Figure 7 shows a collapsible extending seal comprising a sheath 100 of nylon or other suitable material that is placed around the tendon-integrated cable enveloping it to thereby allow the cable to extend beyond the skin and indeed move through and beyond the skin yet remain completely shut-off from the external environment. Since many tendons shorten by only a few centimetres even at maximum contraction (eg flexor tendons to the digits shorten by 7-8 cm while flexors of the wrist shorten by 3-4 cm when the muscles are fully contracted) the sheath 100 in the embodiment of Figure 7 allows for the required extent of movement.

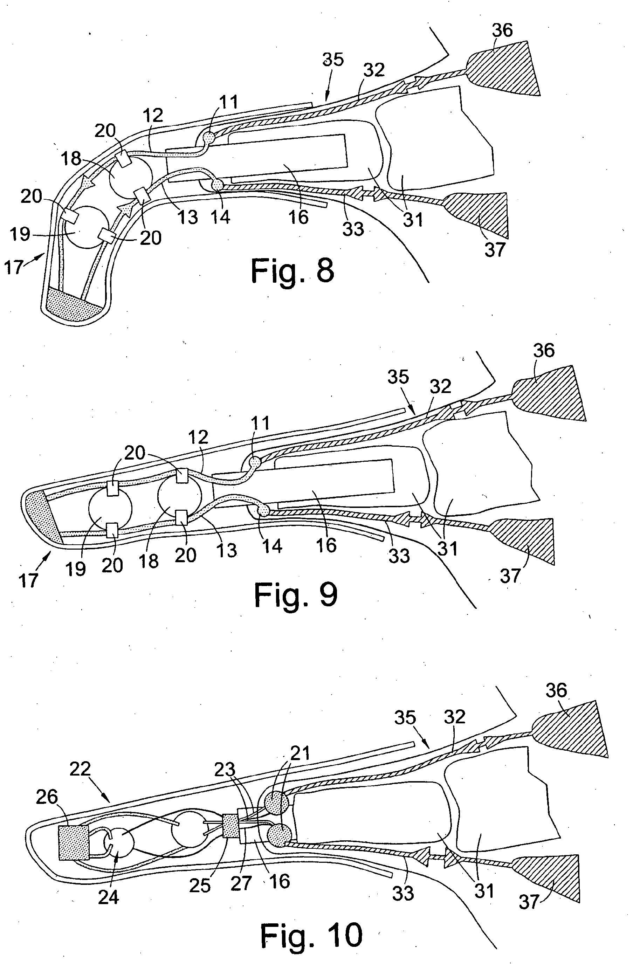

In a fourth embodiment, shown in Figures 8 and 9, the device has first and second connectors or artificial tendons 12, 13. A first connector 12 is connected to a first tendon-fixture 11, the tendon-fixture 11 being connected to the native extendor tendon 32 in the residual limb. A second connector 13 is connected to a second tendon-fixture 14, the tendon-fixture 14 being connected to the native flexor tendon 33 in the residual limb. Preferably the tendon-fixtures 11, 14 are as described in relation to the first to third embodiments.

The device includes a transcutaneous anchor component 16 for anchoring an external prosthesis 17 to the patient's bone. As described for the previous embodiments, the transcutaneous anchor component 16 includes a bone-integrated fixture implanted into the patient's bone 31 and a transcutaneous portion that projects through the patient's skin 35.

As described for the previous embodiments, the connectors 12, 13 may be cables or cords, such as nylon cords. The transcutaneous anchor 16 includes first and second sealed sleeves (not shown in Figures 8 and 9) through which the first and second connectors 12, 13 pass through respectively. The sealed sleeves provide a sealed feed-through for the connectors 12, 13 within the transcutaneous anchor 16, the connectors 12, 13 being able to move back and forth within their respective sleeves and allowing transmission of the force of muscle contraction to the external prosthesis.

The first connector 12 acts as an artificial extendor tendon and the second connector 13 acts as an artificial flexor tendon for the external prosthetic joint 17. The first and second connectors 12, 13 are fixedly coupled at their distal ends to the external prosthesis 17 (in Figures 8 and 9 the external prosthesis is a prosthetic finger). Preferably the external prosthesis 17 includes first and second pulleys 18, 19 located next to one another, along the axis of the prosthesis 17. Each pulley 18, 19 has a first runner 20 located on a side of the pulley adjacent the top of the prosthesis 17 and a second runner 20 located on an opposite side of the pulley, adjacent the underside of the prosthesis 17. The first connector 12 passes through the runners 20 on the top sides of the first and second pulleys 18, 19 and the second connector 13 passes through the runners 20 on the bottom sides of the first and second pulleys 18, 19. The runners 20 may be tubular or cylindrical fixings, their inside surfaces being low-friction surfaces, such that the connectors 12, 13 may slide easily through the runners 20.

Referring to Figure 8, when the muscle 36 to which the native extendor tendon 32 is attached relaxes and the muscle 37 to which the native flexor tendon 33 is attached contracts, the first connector 12 is caused to move distally within its sleeve and the second connector 13 is caused to move proximally within its sleeve, whereby the prosthetic joint 17 is caused to flex.

Referring to Figure 9, when the muscle 36 to which the native extendor tendon 32 is attached contracts and the muscle 37 to which the native flexor tendon 33 is attached relaxes, the first connector 12 is caused to move proximally within its sleeve and the second connector 13 is caused to move distally within its sleeve, whereby the prosthetic joint 17 is caused to extend.

Figures 8 and 9 show a finger prosthetic device having two artificial tendons or connectors 12, 13. An external prosthesis having one artificial tendon may of course be used. In this case, the external prosthesis would be biased in its rest position as either extended or flexed. If the prosthesis were biased in the extended position, then the single artificial tendon would be able to apply the required force to flex the prosthetic joint as required, the prosthetic joint spontaneously returning to the extended position when the force is removed. Similarly, if the prosthesis were biased in the flexed position, the single artificial tendon would be able to apply the required force in order to extend the prosthetic joint, the prosthetic joint spontaneously returning to the flexed position when the force is removed.

In a fifth embodiment, shown in Figure 10, the device uses electrical signals to extend/flex the external prosthetic joint. The device includes a transcutaneous anchor component 16 for anchoring an external prosthesis 22 to the patient's bone. As described for the previous embodiments, the transcutaneous anchor component 16 includes a bone-integrated fixture implanted into the patient's bone 31 and a transcutaneous portion that projects through the patient's skin 35.

The transcutaneous prosthetic device of Figure 10 comprises one or more tendon-fixtures 21 connected to the native tendons in the residual limb. In Figure 10, two tendon-fixtures 21 are shown, one connected to the native extendor tendon 32 and the other connected to the native flexor tendon 33. Each tendon-fixture 21 incorporates a sensor which generates electrical signals in response axial movement of the tendon to which it is connected, due to contraction and/or relaxation of muscle in the residual limb. Sensors which may be used include potentiometers and load cells. A potentiometer can be used to measure axial movement of a tendon or an artificial extension connected thereto. A load cell or strain gauge can be used to measure the force exerted by the tendon.

The sensor in each tendon-fixture 21 is electrically coupled to the external prosthetic device 22 by an electrical connector 23 such as a wire or cable. Preferably each electrical connector 23 passes through the transcutaneous anchor component 16 via a sealed feed-through, such as a ceramic feed-through, the connection being hermetically sealed to provide an effective barrier between the internal and external environments. The electrical connectors 23 are electrically insulated and are embedded in the body of the transcutaneous prosthesis 16, sealed against risk of ingress of microbes to prevent ascending infection from entering the residual limb, which might lead to serious harm for the amputee. The seal around the electrical connectors 23 prevents air or fluids from being able to pass from the external to the internal environment, thus preventing infection.

A single electrical connector may connect the sensor of each tendon-fixture 21 directly to a component in the external prosthesis 22, the electrical connector passing through the transcutaneous anchor component 16 via a sealed feed-through. Alternatively the transcutaneous anchor may have subcutaneous electrical contacts and/or extracutaneous contacts on its surface to which wires may connect, linking the or each sensor to the external prosthesis 22. Preferably the contacts are polished electrical contacts.

The electrical connectors 23 couple the sensors in the tendon-fixtures 21 to one or more electric motors 24 in the external prosthesis 22, preferably via an amplifier 25 to amplify the electrical signals from the sensors. The electric motor(s) 24 drive flexion and/or extension of the prosthetic joint 22. The external prosthesis 22 also includes a battery 26, connected to the electric motor(s) 24 and to the sensors in the tendon-fixtures 21, to supply power thereto. A rechargeable battery may of course be used. Electrical connectors 27, which connect the battery 26 to the sensors, each preferably pass through the transcutaneous anchor component 16 via a sealed feed-through, such as a ceramic feed-through, the connection being hermetically sealed to provide an effective barrier between the internal and external environments.

When a muscle, 36 or 37, in the residual limb contracts or relaxes, a force is applied to the native tendon, 32 or 33, causing the native tendon to move axially, the axial movement being detected by the sensor in the corresponding tendon-fixture 21 to which the native tendon is attached. When axial tendon movement is detected, the sensor generates an electrical signal which is transmitted via the electrical connector 23 to the external prosthesis 22. The electrical signal may provide information to the external prosthesis 22 as to the direction and extent of the tendon movement. Control circuitry may be included in the external prosthesis 22 for controlling the electric motor(s) 24 in response to the signals transmitted from the sensor(s).

Although the figures show finger prostheses, the present invention may of course be used for reconstruction of other distal amputations, such as toes or for other limbs such as hands, feet, arms and legs.

## Claims

1. A transcutaneous prosthetic device which comprises:
(i) a or at least one soft tissue fixture (1, 11, 14, 21) adapted to be fixed to the musculo-tendinous soft tissues (20, 32) of a residual limb;
(ii) a transcutaneous anchor (5, 6, 16) for an external prosthesis that is fixedly coupled, directly or indirectly, to the bone (31) of the residual limb in use, the transcutaneous anchor having or being rigidly coupled to a transcutaneous component having a body which passes through the skin in use;
(iii) at least one transmission means (3, 12, 13, 23), at least part of the transmission means being housed, in use, within said body of the transcutaneous component, the transmission means allowing transmission of one or more signals between the soft tissue fixture and an external prosthesis (22, 40), the signal or signals relating to contraction and/or relaxation of muscle in the residual limb, whereby the external prosthesis can be controlled by contraction and/or relaxation of muscle in the residual limb.

2. A device as claimed in claim 1 wherein the transmission means comprises a connecting means (3, 12, 13, 23) connecting the soft tissue fixture and an external prosthesis in use, at least part of the connecting means being housed, in use, within said body of the transcutaneous component, whereby the external prosthesis can be controlled via the connecting means, by contraction and/or relaxation of muscle in the residual limb.

3. A device as claimed in claim 2, wherein the connecting means comprises a mechanical (3, 12, 13) or an electrical (23) connector.

4. A device as claimed in claim 2 or 3, wherein the connecting means (3, 12, 13, 23) comprises a sealed connection within the transcutaneous component, to provide an effective barrier between the internal and external environments.

5. A device as claimed in claims 2, 3 or 4, wherein the connecting means comprises a connector (3, 12, 13) attached, in use, by its proximal end to the fixture (1, 11, 14) and which allows the mechanical force of muscle contraction to be transmitted to an external prosthesis, the transcutaneous (5, 6) anchor having or being rigidly coupled to a transcutaneous sleeve component (9) into which the connector extends subcutaneously and from which the connector or an extension thereof extends externally from the skin, the sleeve component forming a fluid tight barrier, the subcutaneous part of the connector being slidable back and forth into the transcutaneous sleeve component whereby the external part or extension of the connector correspondingly moves back and forth.

6. A device as claimed in claim 5, wherein the sleeve component (9) further comprises a rotary member (7, 8) and the connector (3) is attached at its distal end to the rotary member to rotate the rotary member (7, 8) when the connector (3) moves back and forth, a or the fluid tight barrier being formed around the rotary member, the movement of the connector being transmitted beyond the sleeve by an extension connector attached to the rotary member (7, 8), or an extension thereof, on the external side of the barrier.

7. A device as claimed in claim 5 or 6, wherein the sleeve component (9) further comprises an extendible and collapsible envelope/ sheath (100) that envelops the external part or extension of the connector.

8. A device as claimed in claim 5, 6 or 7, wherein the connector (3)comprises a cable or cord the surface of which has a non-stick coating or is otherwise adapted to deter tissue adhesion and minimise friction with the surrounding tissues.

9. A device as claimed in claim 5, 6, 7 or 8 wherein the connector (3) is housed in a sheath for a substantial part of its length.

10. A device as claimed in claim 2, 3 or 4, wherein the connecting means comprises an electrical connector (23), the connecting means or the fixture (21) including a sensor which generates an electrical signal in response to contraction and/or relaxation of muscle in the residual limb.

11. A device as claimed in claim 10, wherein the sensor -comprises a potentiometer or a strain guage.

12. A device as claimed in claim 10 or 11, wherein the transcutaneous component has at least one feed-through connection into which the electrical connector or an extension thereof extends subcutaneously and from which the electrical connector or an extension thereof extends externally from the skin in use, the feed-through connection being sealed to provide an effective barrier between the internal and external environments.

13. A device as claimed in claim 10, 11 or 12, wherein the sensor is connectable, via a sealed connection within said body of the transcutaneous component, to a battery (26) in an external prosthesis.

14. A device as claimed in claim 10, 11, 12 or 13, wherein the sensor is connectable, via a sealed connection within said body of the transcutaneous component, to at least one electric motor (24) adapted to drive movement of the external prosthesis.

15. A device as claimed in any preceding claim, wherein the soft tissue fixture (1, 11, 14, 21) is adapted to be integrated into the soft tissue, being of a material or having a surface treatment to encourage in-growth of collagen fibre

## Patentansprüche

1. Transkutane Prothesevorrichtung, die Folgendes umfasst:
(i) eine oder mindestens eine Weichteilbefestigung (1, 11, 14, 21), die so angepasst ist, dass sie an den muskulotendinösen Weichteilen (20, 32) eines Stumpfes befestigt werden kann;
(ii) einen transkutanen Anker (5, 6, 16) für eine Exoprothese, der in Verwendung direkt oder indirekt fest mit dem Knochen (31) des Stumpfes gekoppelt ist, wobei der transkutane Anker eine transkutane Komponente aufweist oder starr mit einer transkutanen Komponente gekoppelt ist, die einen Körper aufweist, der die Haut in Verwendung durchdringt;
(iii) mindestens eine Übertragungsvorrichtung (3, 12, 13, 23), wobei mindestens ein Teil der Übertragungsvorrichtung in Verwendung im Inneren des Körpers der transkutanen Komponente angeordnet ist, wobei die Übertragungsvorrichtung die Übertragung von einem oder mehreren Signalen zwischen der Weichteilbefestigung und einer Exoprothese (22, 40) ermöglicht, wobei das Signal oder die Signale mit Muskelkontraktion und/oder -relaxation im Stumpf in Verbindung steht bzw. stehen, wodurch die Exoprothese durch Muskelkontraktion und/oder -relaxation im Stumpf gesteuert werden kann.

2. Vorrichtung nach Anspruch 1, wobei die Übertragungsvorrichtung eine Verbindungsvorrichtung (3, 12, 13, 23) umfasst, die in Verwendung die Weichteilbefestigung und eine Exoprothese verbindet, wobei wenigstens ein Teil der Verbindungsvorrichtung in Verwendung im Inneren des Körpers der transkutanen Komponente angeordnet ist, wodurch die Exoprothese über die Verbindungsvorrichtung durch Muskelkontraktion und/oder -relaxation im Stumpf gesteuert werden kann.

3. Vorrichtung nach Anspruch 2, wobei die Verbindungsvorrichtung einen mechanischen (3, 12, 13) oder einen elektrischen (23) Verbinder umfasst.

4. Vorrichtung nach Anspruch 2 oder 3, wobei die Verbindungsvorrichtung (3, 12, 13, 23) eine abgedichtete Verbindung im Inneren der transkutanen Komponente umfasst, um eine wirksame Barriere zwischen der internen und externen Umgebung bereitzustellen.

5. Vorrichtung nach den Ansprüchen 2, 3 oder 4, wobei die Verbindungsvorrichtung einen Verbinder (3, 12, 13) umfasst, der in Verwendung an seinem proximalen Ende an der Befestigung (1, 11, 14) befestigt ist und der es ermöglicht, dass die mechanische Kraft der Muskelkontraktion an eine Exoprothese übertragen wird, wobei der transkutane (5, 6) Anker eine transkutane Hülsenkomponente (9) aufweist oder starr mit einer transkutanen Hülsenkomponente verbunden ist, in die sich der Verbinder subkutan erstreckt und von der der Verbinder oder eine Verlängerung desselben sich extern aus der Haut erstreckt, wobei die Hülsenkomponente eine flüssigkeitsdichte Barriere bildet und der subkutane Teil des Verbinders vor und zurück in die transkutane Hülsenkomponente geschoben werden kann, wodurch der externe Teil oder die Verlängerung des Verbinders sich entsprechend vor- und zurückbewegt.

6. Vorrichtung nach Anspruch 5, wobei die Hülsenkomponente (9) ferner ein Drehelement (7, 8) umfasst und der Verbinder (3) an seinem distalen Ende an dem Drehelement befestigt ist, sodass er das Drehelement (7, 8) dreht, wenn der Verbinder (3) sich vor- und zurückbewegt, wobei eine oder die flüssigkeitsdichte Barriere um das Drehelement gebildet ist und die Bewegung des Verbinders durch einen Verlängerungsverbinder, der am Drehelement (7, 8) oder einer Verlängerung desselben befestigt ist, an der externen Seite der Barriere über die Hülse hinaus übertragen wird.

7. Vorrichtung nach Anspruch 5 oder 6, wobei die Hülsenkomponente (9) ferner eine erweiterbare und zusammenfaltbare Ummantelung/Hülle (100) umfasst, die den externen Teil oder die Verlängerung des Verbinders umschließt.

8. Vorrichtung nach Anspruch 5, 6 oder 7, wobei der Verbinder (3) ein Kabel oder eine Leitung umfasst, dessen bzw. deren Oberfläche eine Antihaftbeschichtung aufweist oder anderweitig dafür angepasst ist, Gewebeadhäsion zu verhindern und Reibung mit den angrenzenden Geweben zu minimieren.

9. Vorrichtung nach Anspruch 5, 6, 7 oder 8, wobei ein wesentlicher Teil der Länge des Verbinders (3) in einer Hülle angeordnet ist.

10. Vorrichtung nach Anspruch 2, 3 oder 4, wobei die Verbindungsvorrichtung einen elektrischen Verbinder (23) umfasst und wobei die Verbindungsvorrichtung oder die Befestigung (21) einen Sensor umfasst, der als Reaktion auf Muskelkontraktion und/oder -relaxation im Stumpf ein elektrisches Signal erzeugt.

11. Vorrichtung nach Anspruch 10, wobei der Sensor ein Potentiometer oder einen Dehnungsmesser umfasst.

12. Vorrichtung nach Anspruch 10 oder 11, wobei die transkutane Komponente mindestens eine Durchgangsverbindung aufweist, in die sich in Verwendung der elektrische Verbinder oder eine Verlängerung desselben subkutan erstreckt und aus der sich der elektrische Verbinder oder eine Verlängerung desselben extern aus der Haut erstreckt, wobei die Durchgangsverbindung abgedichtet ist, um eine wirksame Barriere zwischen der internen und externen Umgebung bereitzustellen.

13. Vorrichtung nach Anspruch 10, 11 oder 12, wobei der Sensor über eine abgedichtete Verbindung im Inneren des Körpers der transkutanen Komponente mit einer Batterie (26) in einer Exoprothese verbunden werden kann.

14. Vorrichtung nach Anspruch 10, 11, 12 oder 13, wobei der Sensor über eine abgedichtete Verbindung im Inneren des Körpers der transkutanen Komponente mit mindestens einem Elektromotor (24) verbunden werden kann, der dafür angepasst ist, die Bewegung der Exoprothese anzutreiben.

15. Vorrichtung nach einem der vorstehenden Ansprüche, wobei die Weichteilbefestigung (1, 11, 14, 21) so angepasst ist, dass sie in die Weichteile integriert werden kann, und aus einem Material besteht oder eine Oberflächenbehandlung aufweist, das oder die den Einwuchs von Kollagenfasern begünstigt.

## Revendications

1. Dispositif prothétique transcutané comprenant :
(i) un ou au moins un dispositif de fixation pour tissus mous (1,11,14,21) adaptés pour se fixer aux tissus mous musculo-tendineux (20, 32) d'un moignon ;
(ii) un ancrage transcutané (5, 6, 16) pour prothèse externe accouplée de manière fixe, directement ou indirectement, à l'os (31) du moignon pendant l'utilisation, l'ancrage transcutanée possédant un composant transcutané ou étant solidement accouplé à un composant transcutané présentant un corps qui passe à travers la peau pendant l'utilisation;
(iii) au moins un moyen de transmission (3, 12, 13, 23), au moins une partie du moyen de transmission étant logée, pendant l''utilisation, à l'intérieur dudit corps du composé transcutané, le moyen de transmission permettant la transmission d'un ou plusieurs signaux entre le dispositif de fixation pour tissus mous et une prothèse externe (22, 40), le signal ou les signaux relatifs à la contraction et/ou la relaxation des muscles du moignon, permettant de commander la prothèse externe par la contraction et/ou la relaxation des muscles du moignon.

2. Dispositif selon la revendication 1, où le moyen de transmission comprend un moyen de connexion (3, 12, 13, 23) reliant le dispositif de fixation pour tissus mous et une prothèse externe pendant l'utilisation, au moins une partie du moyen de connexion étant logé, pendant d'utilisation, à l'intérieur dudit corps du composant transcutané, permettant de commander la prothèse externe à l'aide du moyen de connexion, par contraction et/ou relaxation des muscles du moignon.

3. Dispositif selon la revendication 2, où le moyen de connexion comprend un connecteur mécanique (3, 12, 13) ou électrique (23).

4. Dispositif selon les revendications 2 ou 3, où le moyen de connexion (3, 12, 13, 23) comprend une connexion étanche à l'intérieur du composé transcutané, pour fournir une barrière efficace entre les environnements internes et externes.

5. Dispositif selon les revendications 2, 3 ou 4, où le moyens de connexion comprend un connecteur (3, 12, 13) attaché, pendant l'utilisation, par son extrémité proximale au dispositif de fixation (1, 11, 14) et qui permet de transmettre la force mécanique de la contraction musculaire à une prothèse externe, l'ancrage transcutané (5, 6) possédant un composant de manchon transcutané ou étant solidement accouplé à un composant de manchon transcutané (9) dans lequel le connecteur s'étend par voie sous-cutanée et depuis lequel le connecteur ou un rallonge de celui-ci s'étend à l'extérieur de la peau, le composant de manchon formant une barrière étanche au fluide, la partie sous-cutanée du connecteur pouvant coulissant vers l'avant et vers l'arrière dans le composant de manchon transcutané, tandis que la partie externe composante ou la rallonge du connecteur effectue un mouvement de va-et-vient correspondant.

6. Dispositif selon la revendication 5, où le composant de manchon (9) comprend en outre un élément rotatif (7, 8) et le connecteur (3) est attaché à son extrémité distale à l'élément rotatif pour faire pivoter l'élément rotatif (7, 8) lorsque le connecteur (3) effectue un mouvement de va-et-vient, une barrière étanche au fluide ou la barrière étanche au fluide étant formée autour de l'élément rotatif, le mouvement du connecteur étant transmis au-delà du manchon par un connecteur à rallonge attaché à l'élément rotatif (7, 8), ou une rallonge de celui-ci, sur le côté extérieur de la barrière.

7. Dispositif selon les revendications 5 ou 6, où le composant de manchon (9) comprend en outre une enveloppe/une gaine extensible et repliable (100) qui enveloppe la partie externe ou la rallonge du connecteur.

8. Dispositif selon les revendications 5, 6 ou 7, où le connecteur (3) comprend un câble ou un cordon dont la surface présente un revêtement antiadhésif ou est autrement adapté pour empêcher l'adhérence des tissus et minimiser le frottement avec les tissus environnants.

9. Dispositif selon les revendications 5, 6, 7 ou 8, où le connecteur (3) est logé dans une gaine pour une grande partie de sa longueur.

10. Dispositif selon les revendications 2, 3 ou 4, où le moyen de connexion comprend un connecteur électrique (23), le moyen de connexion ou la fixation (21) comprenant un capteur qui génère un signal électrique en réponse à la contraction et/ou la relaxation des muscles du moignon.

11. Dispositif selon la revendication 10, où le capteur comprend un potentiomètre ou une jauge de contrainte.

12. Dispositif selon les revendications 10 ou 11, où le composant transcutané possède a au moins une connexion traversante dans laquelle s'étend le connecteur électrique ou une rallonge de celui-ci par voie sous-cutanée et à partir de laquelle s'étend le connecteur électrique ou une rallonge de celui-ci à l'extérieur de la peau pendant l'utilisation, la connexion traversante étant étanche pour fournir une barrière efficace entre les environnements internes et externes.

13. Dispositif selon les revendications 10, 11 ou 12, où le capteur est raccordable, par le biais d'une connexion étanche à l'intérieur dudit corps du composant transcutané, dans une pile (26) dans une prothèse externe.

14. Dispositif selon les revendications 10, 11, 12 ou 13, où le capteur est raccordable, par le biais d'une connexion étanche à l'intérieur dudit corps du composant transcutané, vers au moins un moteur électrique (24) adapté pour entraîner le mouvement de la prothèse externe.

15. Dispositif selon l'une quelconque des revendications précédentes, où le dispositif de fixation pour tissus mous (1, 11, 14, 21) est adapté pour s'intégrer dans les tissus mous, étant d'un matériau ou ayant un traitement de surface encourageant la croissance interne de fibres de collagène.
